# EUROPÄISCHE PATENTSCHRIFT

(11) **EP 1 974 881 B1**
(45) Veröffentlichungstag und Bekanntmachung des Hinweises auf die Patenterteilung: **23.10.2013**
(21) Anmeldenummer: 08004884.6
(22) Anmeldetag: 15.03.2008
(51) Int. Cl.: B27B 19/00, A61B 17/14

(54) **Anordnung mit einer Sägeblattaufnahme für eine Oszillationssäge**
Device with a saw blade fixture for an oscillation saw
Agencement doté d'un moyeu pour une scie à oscillations

(30) Priorität: 30.03.2007 DE 102007015836; 31.07.2007 DE 102007036322
(43) Veröffentlichungstag der Anmeldung: 01.10.2008
(73) Patentinhaber: WSEngineering GmbH & Co. KG, 78054 Villingen-Schwenningen (DE)
(72) Erfinder: Lay, Norbert, Dr., 71149 Bondorf (DE)
(74) Vertreter: Herden, Andreas F.

(56) Entgegenhaltungen:
- DE-A1-102004 050 799
- US-A- 5 309 805
- US-A1- 2002 069 535
- US-A1- 2004 138 670

## Beschreibung

### Gebiet der Erfindung

Die Erfindung betrifft eine Sägeblattaufnahme für eine Oszillationssäge mit einem Sägeblatt.

Oszillationssägen sind aus der Praxis bekannt. Bei derartigen Werkzeugen ist ein Sägeblatt auf einer Antriebswelle befestigt und wird in oszillierende Schwingung versetzt. Mit Oszillationssägen können auch auf engstem Raum Schlitze gesägt werden, insbesondere kann mit einem solchen Werkzeug bis in Ecken hinein gesägt werden. In der Medizintechnik werden Oszillationssägen auch verwendet, um Gipsverbände aufzusägen, ohne die Haut des Patienten zu verletzen. Dieses Prinzip beruht darauf, dass Oszillationssägen einen zumeist sehr geringen Hub haben, so dass weiches Material wie die Haut des Patienten mit der Säge mitschwingen kann und dadurch nicht oder nur wenig beschädigt wird.

Aus der DE 101 64 081 ist ein Oszillationswerkzeug bekannt, welches einen Ausgleichsabschnitt zum Auswuchten aufweist. Nachteilig an diesem Sägeblatt ist, dass der Auswuchtabschnitt fest mit dem restlichen Sägeblatt verbunden ist und so beim Austausch des Sägeblattes mit ausgetauscht werden muss.

Weiter ist bei diesem Sägeblatt die Befestigungsöffnung mit in das Sägeblatt integriert. Da die Befestigungsöffnung hohe Kräfte aufnehmen muß, müssen hochwertige Materialien verwendet werden, um einen Verschleiß der Befestigungsöffnung zu verhindern. Eine entsprechend teure Ausgestaltung des Sägeblattes ist daher erforderlich.

Das Dokument DE 102004050799 A1 zeigt ein mittels einer Schraube befestigbares Wendeschneidblatt.

### Aufgabe der Erfindung

Der Erfindung liegt demgegenüber die Aufgabe zugrunde, ein System bereitzustellen, bei welchem sich der Austausch des Sägeblatts auf eine möglichst kleine und preiswerte Komponente beschränkt. Insbesondere soll der Materialaufwand beim notwendigen Austausch des Sägeblatts verringert werden.

Eine weitere Aufgabe der Erfindung ist es, Schwingungen der Antriebswelle eines Oszillationswerkzeugs zu reduzieren.

Eine weitere Aufgabe der Erfindung ist es, eine besonders einfache Austauschbarkeit des Sägeblatts zu ermöglichen.

Weiter ist es Aufgabe der Erfindung, die Ankopplung des Sägeblatts an die Antriebswelle möglichst starr auszubilden, insbesondere durch Verbiegungen und Schwingungen bedingte Reduzierungen der Sägeleistung zu verringern.

### Zusammenfassung der Erfindung

Die Aufgabe der Erfindung wird durch eine Anordnung mit einem Sägeblatt und einer Sägeblattaufnahme nach Anspruch 1 erreicht.

Bevorzugte Ausführungsformen und Weiterbildungen der Erfindung sind den jeweiligen Unteransprüchen zu entnehmen.

Die Erfindung betrifft eine Anordnung mit einem Sägeblatt und einer Sägeblattaufnahme für eine Oszillationssäge. Dabei weist die Sägeblattaufnahme Mittel zur Befestigung an der Antriebswelle eines Oszillationssägewerkzeugs und Mittel zur Aufnahme eines Sägeblatts auf.

Die Sägeblattaufnahme ist also als eine Art Adapter ausgebildet, welcher einerseits an einer Oszillationssäge befestigt wird und andererseits die Aufnahme eines Sägeblatts ermöglicht. Das Sägeblatt ist dadurch nicht direkt an der Antriebswelle des Oszillationssägewerkzeugs befestigt.

Gemäß der Erfindung weist das Sägeblatt einen als Aufnahmeabschnitt ausgebildeten abgewinkelten Basisabschnitt auf.

Der Erfinder hat herausgefunden, dass ein abgewinkeltes Sägeblatt einfach und sicher in einem Adapter eingespannt werden kann. Dabei wird vorzugsweise der abgewinkelte Basisabschnitt in der Sägeblattaufnahme eingespannt. Gegenüber gerade ausgebildeten Sägeblättern liefert die Erfindung eine wesentlich stabilere Befestigung, bei welcher es in der Regel nicht zu Bewegungen des eingespannten Sägeblattes relativ zur Aufnahme kommt, welche sowohl zur Beschädigung der Aufnahme als auch des Sägeblattes führen können. Die Verwendung der Sägeblattaufnahme ermöglicht eine materialsparendere und billigere Bereitstellung von Sägeblättern.

So kann ein im Wesentlichen einstückig ausgebildetes Sägeblatt verwendet werden, bei welchem insbesondere auf angeschweißte Verstärkungsteile an der Aufnahmestelle verzichtet werden kann.

Gemäß der Erfindung bildet der abgewinkelte Basisabschnitt mit dem restlichen Sägeblatt einen stumpfen Winkel, insbesondere einen Winkel von 130 bis 140°. So wird die Bruchgefahr des Sägeblatts reduziert.

Bei einer Weiterbildung der Erfindung weist der Basisabschnitt zumindest eine Aussparung auf.

Die Aussparung ermöglicht beispielsweise das Hindurchführen einer Schraube, um mit einem Spannelement das Sägeblatt einspannen zu können.

Bei einer Weiterbildung der Erfindung dient die Aussparung als Formschluss in der Ebene des abgewinkelten Basisabschnitts. Insbesondere ist die Aussparung im Wesentlichen rechteckig ausgebildet, wobei die Kanten der Aussparung zumindest teilweise einen Übergang aufweisen. Durch den vorzugsweise als Radius ausgebildeten Übergang wird die Neigung zur Rissbildung im Bereich der Aussparung zumindest reduziert.

Die Aussparung ist vorzugsweise rechteckig und am oberen Ende des Basisabschnittes offen ausgebildet, um das Sägeblatt in die Sägeblattaufnahme einlegen zu können, ohne ein Spannelement komplett abschrauben zu müssen.

Der Basisabschnitt hat vorzugsweise eine Länge zwischen 5 und 25 mm, besonders bevorzugt zwischen 10 und 20 mm.

Bei einer Weiterbildung der Erfindung ist der abgewinkelte Basisabschnitt mindestens 2, vorzugsweise mindestens 2,5 cm von der Mittelachse der Antriebswelle des Oszillationssägewerkzeugs beabstandet.

Die Sägeblattaufnahme weist somit einen Verlängerungsabschnitt auf, wodurch eine kürzere Ausbildung des Sägeblatts bei gleichem Hub ermöglicht wird. So ist es möglich, Sägeblätter mit einer Länge zwischen 35 bis 110 mm, bevorzugt zwischen 45 bis 80 mm zu verwenden.

Die Mittelachse der Antriebswelle wird in der Regel durch eine Bohrung oder sonstige im Wesentlichen symmetrische Aussparung in der Sägeblattaufnahme definiert, welche als Durchgangsöffnung für eine Schraube oder ein ähnliches Befestigungselement vorgesehen ist, um die Sägeblattaufnahme am Oszillationssägewerkzeug zu befestigen.

Die Halterung kann ein im Wesentlichen dem Sägeblatt gegenüber liegendes Gegengewicht aufweisen.

Das Sägeblatt wird also in der Halterung befestigt, welche ihrerseits an das Werkzeug angeschraubt wird. Da die Halterung mehrfach verwendet werden kann, können für die Halterung hochwertige Materialien verwendet werden und das Sägeblatt muss, da es nicht direkt mit der Antriebswelle verbunden ist, nicht mehr derart hohe lokale Kräfte aushalten.

Bei einer Weiterbildung der Erfindung liegt der Schwerpunkt der Sägeblattaufnahme, also der Aufnahme insbesondere ohne ein eingesetztes Sägeblatt, in axialer Richtung zwischen der Oberseite des Gegengewichtes und der Unterseite des Sägeblatts. So wird eine Auswuchtung der Aufnahme auch in axialer Richtung ermöglicht.

Weiter weist der Basisabschnitt bei einer Weiterbildung der Erfindung eine Aussparung auf.

Die Aussparung kann zum einen dazu dienen, um eine Schraube einer Klemmvorrichtung durch das Sägeblatt zu führen. Zum anderen kann die Aussparung einen Anschlag für eine formschlüssige Verbindung bilden.

Der Basisabschnitt weist vorzugsweise eine Länge zwischen 5 und 25 mm und besonders bevorzugt zwischen 10 und 20 mm auf.

Die Kante der Abwinklung zwischen Basisabschnitt und restlichem Sägeblatt ist zur Erhöhung der Festigkeit verrundet und hat vorzugsweise einen Radius von 0,5 bis 6 mm und besonders bevorzugt von 1,5 bis 3,5 mm.

Bei einer weiteren Ausführungsform der Erfindung ist eine Innenkante mit einem Übergang zwischen Basisblatt und restlichem Sägeblatt vorgesehen.

Bei einer Weiterbildung der Erfindung weist das Sägeblatt 8 bis 35, bevorzugt 13 bis 25 Zähne pro Zoll (teeth per inch) auf. Mit einem derartig ausgebildeten Sägeblatt, welches vorzugsweise eine Wellenschränkung aufweist, lassen sich sowohl Metalle als auch Holz bearbeiten.

Bei einer bevorzugten Ausführungsform der Erfindung weist das Sägeblatt eine angeschweißte Schneide aus hochlegiertem Stahl, insbesondere aus HSS-Stahl oder Super-HSS-Stahl auf. Derartige Schneiden sind von anderen Sägeblättern bekannt. Es handelt sich zumeist um Schneiden, welche mittels einer Laserschweißnaht an einem vorzugsweise aus Werkzeugstahl oder Federstahl ausgebildeten Sägeblatt befestigt sind. Derartige Sägeblätter haben den Vorteil hoher Flexibilität bei gleichzeitig hoher Standzeit der Schneiden. Insbesondere neigen die Schneiden nicht, wie es bei einstückig ausgebildeten Sägeblättern oft der Fall ist, zum Abbrechen. Weiter umfasst die Erfindung eine Oszillationssäge mit einer Sägeblattaufnahme und/oder einem erfindungsgemäßen Sägeblatt.

### Kurzbeschreibung der Zeichnungen

Die Erfindung soll anhand eines schematischen Ausführungsbeispiels Bezug nehmend auf die Zeichnungen Fig. 1 bis Fig. 6 näher erläutert werden.
- Fig. 1: zeigt eine nicht erfindungsgemäße schematische perspektivische Ansicht einer Sägeblattaufnahme.
- Fig. 2: zeigt nicht erfindungsgemäße eine schematische Seitenansicht.
- Fig. 3: zeigt eine nicht erfindungsgemäße schematische Draufsicht.
- Fig. 4: zeigt schematisch ein Ausführungsbeispiel für ein nicht erfindungsgemäßes Sägeblatt.
- Fig. 5: zeigt schematisch eine nicht erfindungsgemäße Ausführungsform einer Sägeblattaufnahme nebst eingespanntem Sägeblatt, bei welcher kein Gegengewicht vorgesehen ist.
- Fig. 6: zeigt eine schematische Seitenansicht der in Fig. 5 dargestellten Anordnung.
- Fig. 7: zeigt ein erfindungsgemäßes Ausführungsbeispiel einer Sägeblattaufnahme nebst eingespanntem Sägeblatt in einer Schnittansicht.

### Detaillierte Beschreibung der Zeichnungen

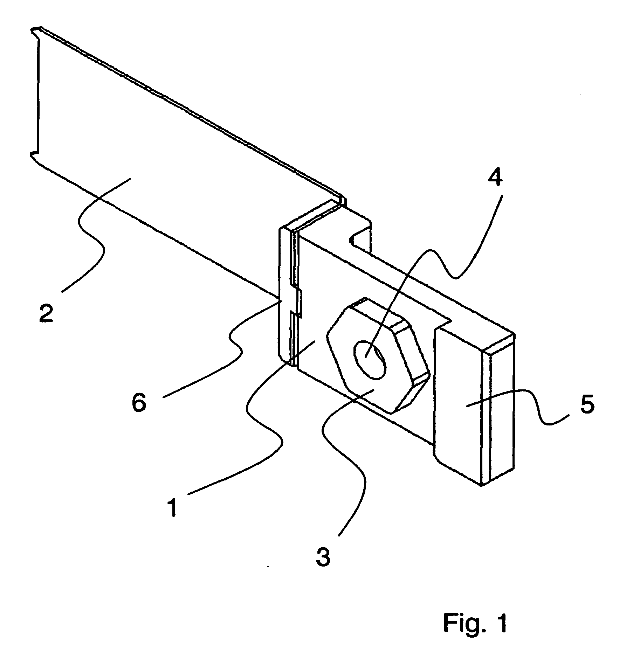

Bezug nehmend auf Fig. 1, welche eine schematische perspektivische Ansicht eines Ausführungsbeispiels einer nicht erfindungsgemäßen Sägeblattaufnahme 1 mit eingesetztem Sägeblatt 2 zeigt, sollen die wesentlichen Elemente der Sägeblattaufnahme näher erläutert werden.

Die Sägeblattaufnahme 1 umfasst ein Sechskant-förmiges Kopplungselement 3 zur formschlüssigen Verbindung mit einer Antriebswelle (nicht dargestellt). Das Kopplungselement ist in diesem Ausführungsbeispiel ein 19 mm Sechskant, welcher einstückig mit der restlichen Sägeblattaufnahme 1 verbunden ist. Über eine Bohrung 4 kann die Sägeblattaufnahme 1 an eine Oszillationssäge (nicht dargestellt) geschraubt werden.

Alternativ ist eine kraftschlüssige Verbindung, vorzugsweise mittels einer Schraube, vorgesehen (nicht dargestellt).

Weiter umfasst die Sägeblattaufnahme 1 ein Spannelement 6, mit welchem das Sägeblatt 2 an der Sägeblattaufnahme befestigt werden kann. Dazu ist das Sägeblatt 2 in einem Winkel von 90° abgewinkelt. Um das Werkzeug bezogen auf die Achse der Bohrung 4 zu wuchten, weist die Sägeblattaufnahme 1 ein Gegengewicht 5 auf, welches dem Spannelement 6 gegenüberliegt. Das Gegengewicht 5 kompensiert im Wesentlichen das Gewicht des Spannelements 6 und/oder des Sägeblatts 2.

In diesem Ausführungsbeispiel ist das Gegengewicht 5 mit der Sägeblattaufnahme 1 einstückig ausgebildet.

Um das Sägeblatt zu entfernen, kann das Spannelement 6 mittels einer Schraube (nicht zu sehen) gelockert oder abgeschraubt werden, um das Sägeblatt 2 herauszunehmen.

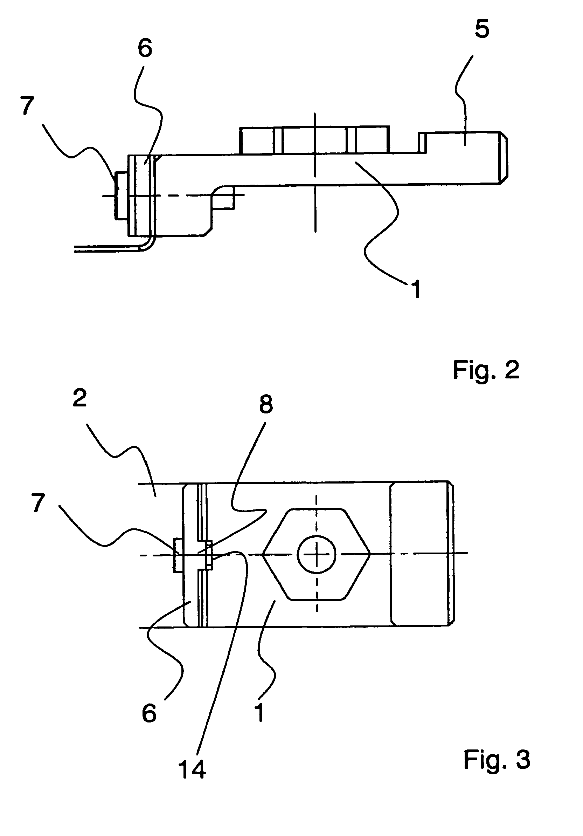

Fig. 2 zeigt eine schematische Seitenansicht der in Fig. 1 dargestellten Sägeblattaufnahme. Zu erkennen ist insbesondere die Schraube 7, mit welcher das Spannelement 6 an die restliche Sägeblattaufnahme 1 geschraubt wird. Die Sägeblattaufnahme ist in diesem Ausführungsbeispiel also dreistückig ausgebildet und umfasst eine Schraube 7, das Spannelement 6 sowie die restliche Sägeblattaufnahme 1. Das Gegengewicht 5 erstreckt sich in diesem Ausführungsbeispiel nach oben, wohingegen sich das Spannelement nach unten erstreckt. Der Schwerpunkt der Sägeblattaufnahme liegt also in axialer Richtung zwischen der oberen Fläche des Gegengewichts 5 und der unteren Fläche des Spannelements 6.

Fig. 3 zeigt eine schematische Draufsicht auf eine Sägeblattaufnahme 1. Zu erkennen ist auch hier die Schraube 7, mit welcher das Spannelement 6, welches im Wesentlichen die gleiche Breite und Höhe wie die gegenüberliegende Anschlagfläche der restlichen Sägeblattaufnahme 1 hat, befestigt werden kann.

Neben einer Verbindung durch Klemmen sieht die Erfindung auch eine formschlüssige Fixierung des Sägeblatts 2 vor.

Dazu weist in diesem Ausführungsbeispiel das Spannelement 6 einen Fortsatz auf, der mit einer in der Sägeblattaufnahme 1 eingebrachten Aussparung 14 korrespondiert. Das Sägeblatt 2, welches ebenfalls eine Aussparung aufweist, wird so zumindest in horizontaler Richtung formschlüssig gehalten.

Eine alternative Ausführungsform der Erfindung sieht vor, den Fortsatz 8 an der Sägeblattaufnahme 1 anzuordnen und das Spannelement 6 mit einer korrespondierenden Aussparung zu versehen (nicht dargestellt).

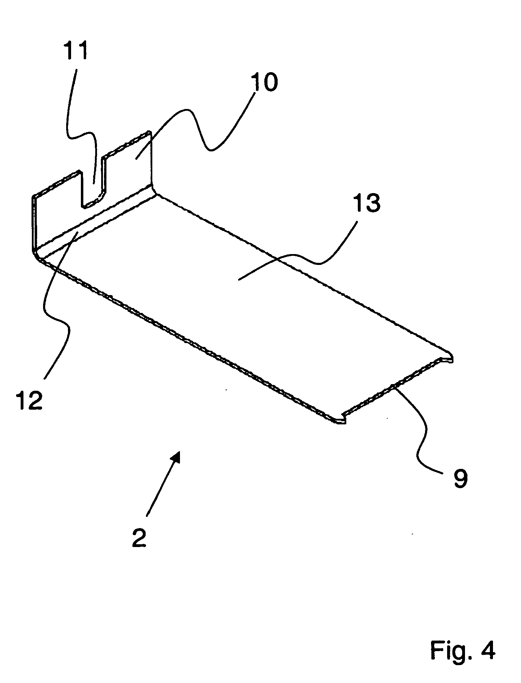

Fig. 4 zeigt eine schematische, perspektivische Ansicht eines nicht erfindungsgemäßen Sägeblatts 2. Das Sägeblatt weist eine Basis 10 auf, welche mit dem restlichen Sägeblatt 13 einen Winkel bildet. In diesem Ausführungsbeispiel beträgt der Winkel etwa 90°.

Gemäß der Erfindung ist aber auch vorgesehen, einen Winkel von über 90°, insbesondere einen Winkel zwischen 90 und 150° bei entsprechend abgeänderter Sägeblattaufnahme zu verwenden. Ein derartiger stumpfer Winkel hat den Vorteil einer besseren Haltbarkeit des Sägeblatts, insbesondere wird an der Innenkante 12 die Neigung zu Kerbbildungen reduziert.

Weiter weist das Sägeblatt 2 vorne eine Schneidkante 9 auf, deren Zähne nur an den beiden Ecken angedeutet sind.

Die Innenkante 12 ist in diesem Ausführungsbeispiel gerundet und hat einen Innenradius von 3 mm.

Um das Sägeblatt formschlüssig zumindest in horizontaler Richtung in der Sägeblattaufnahme (nicht dargestellt) einspannen zu können, weist das Sägeblatt 2 in der Basis 10 eine Aussparung 11 auf. Die Aussparung 11 korrespondiert im Wesentlichen mit dem Fortsatz des Spannelements (nicht dargestellt).

In diesem Ausführungsbeispiel ist die Aussparung 11 oben offen. So kann das Sägeblatt bereits nach Lockern des Spannelements entnommen werden, ohne dass das Spannelement abgeschraubt werden muss.

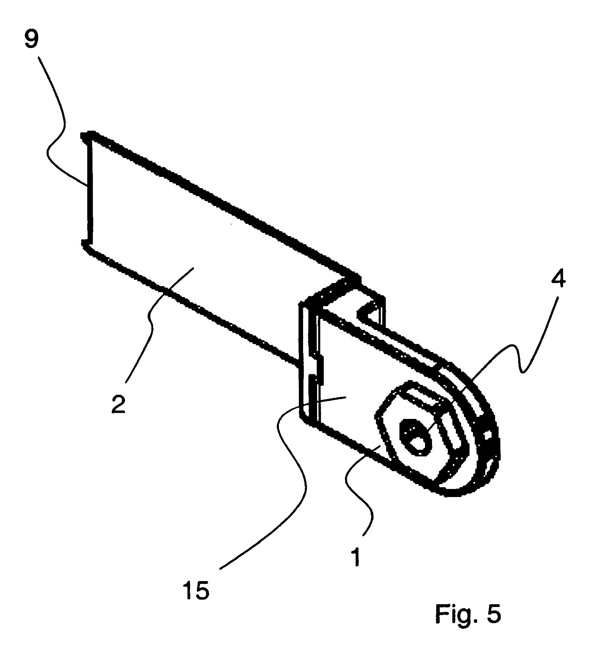

Fig. 5 zeigt eine alternative Ausführungsform einer Anordnung mit einem Sägeblatt 2 und einer Sägeblattaufnahme 1. Diese Anordnung entspricht im Wesentlichen bis auf die nachfolgend dargestellten Unterschiede den zuvor beschriebenen Ausführungsbeispielen.

Die Anordnung weist eine Sägeblattaufnahme 1 und ein Sägeblatt 2 auf.

Im Unterschied zu den zuvor dargestellten Ausführungsbeispielen weist die Sägeblattaufnahme 1 kein Gegengewicht auf.

Vielmehr weist die Sägeblattaufnahme 1 einen Verlängerungsabschnitt 15 auf, welcher eine kürzere Ausbildung des Sägeblatts 2 ermöglicht. Das Sägeblatt 2 ist dadurch zwischen 20 und 40 mm von der Bohrung 4, welche mit der Mittelachse der Antriebswelle (nicht dargestellt) übereinstimmt, beabstandet.

Die Schneide 9 des Sägeblatts 2 besteht aus einer lasergeschweißten Super-HSS-Schneide.

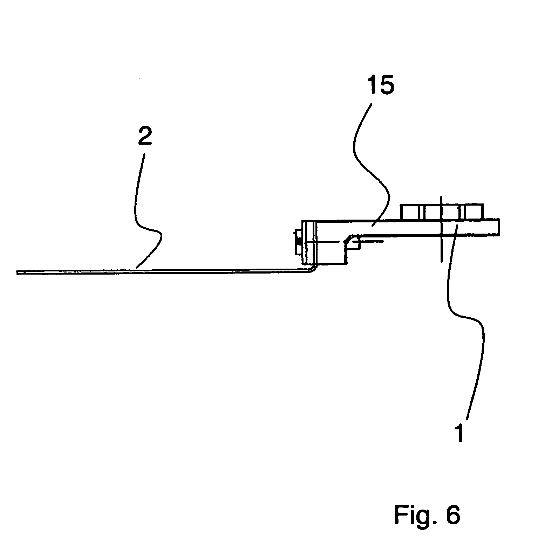

Fig. 6 zeigt das in Fig. 5 dargestellte Ausführungsbeispiel in der Seitenansicht.

Zu erkennen ist auch hier insbesondere der Verlängerungsabschnitt 15 der durch eine langgestreckte Ausbildung der Sägeblattaufnahme 1 erreicht wird, und welcher eine kürzere Ausbildung des Sägeblatts 2 bei gleichbleibendem Hub ermöglicht.

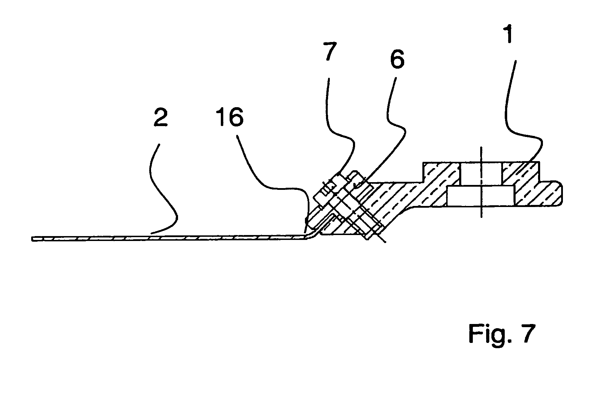

Fig. 7 zeigt ein erfindungsgemäßes Ausführungsbeispiel einer Sägeblattaufnahme 1 mit eingespanntem Sägeblatt 2 in der Schnittaufsicht.

In diesem Ausführungsbeispiel ist das Sägeblatt nicht um 90° sondern um etwa 135° abgewinkelt. Es hat sich gezeigt, dass ein stumpfer Winkel die Bruchgefahr des Sägeblatts reduziert. Zur weiteren Reduzierung der Bruchgefahr weist die Innenkante 16 zwischen Basisabschnitt und restlichem Sägeblatt einen Übergang auf, hier in Form eines Radius zwischen 2 und 5 mm.

Die Sägeblattaufnahme 1 inklusive des Spannelements 6 besteht aus einer hochfesten Aluminiumlegierung. Zur Befestigung des Spannelements 6 wird eine hochfeste 12.9 Schraube verwendet.

### Bezugszeichenliste

- 1: Sägeblattaufnahme
- 2: Sägeblatt
- 3: Kopplungselement
- 4: Bohrung
- 5: Gegengewicht
- 6: Spannelement
- 7: Schraube
- 8: Fortsatz
- 9: Schneide
- 10: Basis
- 11: Aussparung
- 12: Innenkante
- 13: restliches Sägeblatt
- 14: Aussparung
- 15: Verlängerungsabschnitt
- 16: Innenkante

## Patentansprüche

1. Anordnung mit einem Sägeblatt (2) und einer Sägeblattaufnahme (1) für eine Oszillationssäge, wobei die Sägeblattaufnahme (1) Mittel zur Befestigung (3) an der Antriebswelle eines Oszillationssägewerkzeugs und Mittel zur Aufnahme des Sägeblatts (2) aufweist, wobei das Sägeblatt (2) einen als Aufnahmeabschnitt ausgebildeten abgewinkelten Basisabschnitt (10) aufweist, welcher in die Sägeblattaufnahme (1) mit einem Spannelement (6) eingespannt ist, **dadurch gekennzeichnet, dass** der abgewinkelte Basisabschnitt (10) mit dem restlichen Sägeblatt (2) einen stumpfen Winkel bildet.

2. Anordnung mit einem Sägeblatt (2) und einer Sägeblattaufnahme (1) nach einem der vorstehenden Ansprüche, **dadurch gekennzeichnet, dass** das der Basisabschnitt (10) eine Länge zwischen 5 und 25 mm, bevorzugt zwischen 10 und 20 mm aufweist.

3. Anordnung mit einem Sägeblatt (2)und einer Sägeblattaufnahme (1) nach einem der vorstehenden Ansprüche, **dadurch gekennzeichnet, dass** das Sägeblatt (2) eine Länge von 35 bis 110 mm, bevorzugt von 45 bis 80 mm hat.

4. Anordnung mit einem Sägeblatt (2) und einer Sägeblattaufnahme (1) nach einem der vorstehenden Ansprüche, **dadurch gekennzeichnet, dass** der abgewinkelte Basisabschnitt (10) mindestens 2, vorzugsweise mindestens 2,5 cm von der Mittelachse der Antriebswelle beabstandet ist.

5. Anordnung mit einem Sägeblatt (2) nach einem der vorstehenden Ansprüche **dadurch gekennzeichnet, dass** die Innenkante (12) des Sägeblatts (2) zwischen Basisabschnitt und restlichem Sägeblatt (2) einen Radius von 0,5 bis 6 mm, bevorzugt von 1,5 bis 3,5 mm hat.

6. Anordnung mit einem Sägeblatt (2) nach einem der vorstehenden Ansprüche, **dadurch gekennzeichnet, dass** das Sägeblatt (2) eine angeschweißte Schneide (9) aus hochlegiertem Stahl, insbesondere HSS-Stahl aufweist.

7. Oszillationssäge, umfassend eine Anordnung mit einem Sägeblatt (2) nach einem der vorstehenden Ansprüche.

## Claims

1. Arrangement having a saw blade (2) and a saw blade receiver (1) for an oscillating saw, wherein the saw blade receiver (1) has means for attachment (3) to the drive shaft of an oscillating saw tool and means for receiving the saw blade (2), wherein the saw blade (2) has an angled base portion (10) which is formed as a receiving portion and which is clamped into the saw blade receiver (1) by a clamping element (6), **characterised in that** the angled base portion (10) forms an obtuse angle with the rest of the saw blade (2).

2. Arrangement having a saw blade (2) and a saw blade receiver (1) as claimed in any one of the preceding claims *[sic]*, **characterised in that** the base portion (10) has a length of between 5 and 25 mm, preferably between 10 and 20 mm.

3. Arrangement having a saw blade (2) and a saw blade receiver (1) as claimed in any one of the preceding claims, **characterised in that** the saw blade (2) has a length of from 35 to 110 mm, preferably from 45 to 80 mm.

4. Arrangement having a saw blade (2) and a saw blade receiver (1) as claimed in any one of the preceding claims, **characterised in that** the angled base portion (10) is spaced at least 2, preferably at least 2,5 cm from the centre axis of the drive shaft.

5. Arrangement having a saw blade (2) as claimed in any one of the preceding claims, **characterised in that** the inner edge (12) of the saw blade (2) between the base portion and the rest of the saw blade (2) has a radius of from 0,5 to 6 mm, preferably from 1,5 to 3,5 mm.

6. Arrangement having a saw blade (2) as claimed in any one of the preceding claims, **characterised in that** the saw blade (2) has a welded-on blade (9) made from high-alloy steel, in particular HSS steel.

7. Oscillating saw comprising an arrangement having a saw blade (2) as claimed in any one of the preceding claims.

## Revendications

1. Dispositif avec une lame de scie (2) et un support (1) de lame de scie pour une scie oscillante, le support (1) de lame de scie comportant des moyens de fixation (3) sur l'arbre d'entraînement d'un outil à scie oscillante et des moyens de support de la lame de scie (2), ladite lame de scie (2) présentant une partie de base (10) coudée réalisée comme partie de maintien, laquelle est serrée par un élément de serrage (6) dans le support (1) de lame de scie, **caractérisé en ce que** la partie de base (10) coudée forme un angle obtus avec le reste de la lame de scie (2).

2. Dispositif avec une lame de scie (2) et un support (1) de lame de scie selon la revendication 1, **caractérisé en ce que** la partie de base (10) a une longueur comprise entre 5 et 25 mm, de préférence entre 10 et 20 mm.

3. Dispositif avec une lame de scie (2) et un support (1) de lame de scie selon l'une des revendications précédentes, **caractérisé en ce que** la lame de scie (2) a une longueur comprise entre 35 et 110 mm, de préférence entre 45 et 80 mm.

4. Dispositif avec une lame de scie (2) et un support (1) de lame de scie selon l'une des revendications précédentes, **caractérisé en ce que** la partie de base (10) coudée est espacée d'au moins 2 cm, de préférence d'au moins 2,5 cm de l'axe médian de l'arbre d'entraînement.

5. Dispositif avec une lame de scie (2) selon l'une des revendications précédentes, **caractérisé en ce que** le bord intérieur (12) de la lame de scie (2) a un rayon compris entre 0,5 et 6 mm, de préférence entre 1,5 et 3,5 mm entre la partie de base et le reste de la lame de scie (2).

6. Dispositif avec une lame de scie (2) selon l'une des revendications précédentes, **caractérisé en ce que** la lame de scie (2) présente un tranchant (9) soudé en acier hautement allié, en particulier en acier HSS.

7. Scie oscillante, comprenant un dispositif avec une lame de scie (2) selon l'une des revendications précédentes.
